(19)

**Europäisches Patentamt**

**European Patent Office**

**Office européen des brevets**

(11) **EP 4 414 378 A1**

(12) **EUROPEAN PATENT APPLICATION**

(43) Date of publication:
**14.08.2024 Bulletin 2024/33**

(21) Application number: **23156132.5**

(22) Date of filing: **10.02.2023**

(51) International Patent Classification (IPC):
*C07K 14/44* (2006.01)   *A61K 38/16* (2006.01)
*A61P 37/00* (2006.01)   *A61P 25/28* (2006.01)

(52) Cooperative Patent Classification (CPC):
**A61P 37/00; A61K 38/16; A61P 25/28; C07K 14/44**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Ustav organicke chemie a biochemie
AV CR, v.v.i.
166 10 Praha 6 (CZ)**

(72) Inventors:
• **Zoll, Sebastian
Praha (CZ)**
• **Sülzen, Hagen
Praha (CZ)**

(74) Representative: **Harber IP s.r.o.
Dukelskych hrdinu 567/52
17000 Praha 7 (CZ)**

Remarks:
The complete document including Reference
Table(s) and the Sequence Listing(s) can be
downloaded from the EPO website

(54) **PROTEIN FOR USE IN THE TREATMENT OF COMPLEMENT DYSREGULATION DISORDERS**

(57)    The present invention relates to a thermostable ISG65 mutant derived from the extracellular domain of the surface protein ISG65 (invariant surface glycoprotein 65(Tbg.972.2.1600)) of the human parasite *Trypanosoma brucei gambiense.* The thermostable ISG65 mutant shows enhanced thermostability in comparison with the wild type extracellular domain of ISG65 and is useful in the treatment of complement dysregulation diseases.

EP 4 414 378 A1

**Description**

Field of Art

The present invention relates to a protein for use in the treatment of complement dysregulation disorders.

Background Art

The complement cascade is a part of the innate immune system which complements the ability of antibodies and phagocytes to clear microbes and damaged cells and promote inflammation. The complement cascade is activated by three biochemical pathways: the classical pathway, the alternative pathway and the lectin pathway. The alternative pathway is responsible for the majority of terminal pathway activation. The alternative pathway is continuously activated at a low level, due to spontaneous hydrolysis of mildly unstable C3, generating C3b. C3b attaches to cell surfaces where it forms the nidus for formation of the C3 and C5 concertases which eventually lead to assembly of the membrane attack complex in the terminal pathway.

Excessive activation of complement proteins is often discovered to be the reason for many diseases. These include e.g. autoimmune diseases, Alzheimer's syndrome, schizophrenia, atypical hemolytic-uremic syndrome, angioedema, macular degeneration, and Crohn's disease (Tichaczek-Goska D.: Adv Clin Exp Med 2012 Jan-Feb;21(1):105-14). Atypical hemolytic uremic syndrome (aHUS), C3 glomerulopathy (C3G), and paroxysmal nocturnal hemoglobinuria (PNH) are prototypical disorders of complement dysregulation (Wong E.K.S. and Kavangh D.: Semin Immunopathol 2018 Jan;40(1):49-64. doi: 10.1007/s00281-017-0663-8).

Mutations in the thioester domain (TED) of human C3b or in the C3b binding domain of human factor H, a negative regulator of the complement cascade, result in a loss of cellular self-recognition, uncontrolled lysis of red blood cells and consequently kidney failure. Treatment of these diseases currently requires, Eculizumab, a monoclonal antibody which targets C5. Due to the prohibitive cost of this drug, cheaper alternatives are much needed.

The application EP 22192040.8 (not publicly available on the filing date of this application) discloses proteins based on native ISG65 protein sequence for use in the treatment of complement dysregulation diseases.

Summary of the Invention

The extracellular domain of the surface protein ISG65 (invariant surface glycoprotein 65 (Tbg.972.2.1600)) of the human parasite *Trypanosoma brucei gambiense* is a complement receptor which targets the complement cascade at several critical intervention points. The extracellular domain of ISG65 binds to native C3, to its reactive activation products C3b and C3(H$_2$O), and to C3d, but not to C3c. Addition of the extracellular domain of ISG65 to human serum results in a concentration-dependent decrease in haemolysis of erythrocytes following the activation of the alternative, but not the classical pathway nor the lecithin pathway.

C3b serves as a central hub in the complement cascade and therefore its inhibition displays a more comprehensive approach for treatment of complement dysregulations than is targeting several downstream effector pathways (e.g. C5 or C9 polymerization). Also, unlike monoclonal antibodies, the ISG65 extracellular domain targets several interaction partners within the cascade, which potentially increases its potency.

For practical use, it is necessary to develop proteins with a high stability, allowing to produce and store medical products. The present invention aims at providing variants based on ISG65 sequence which have an improved stability.

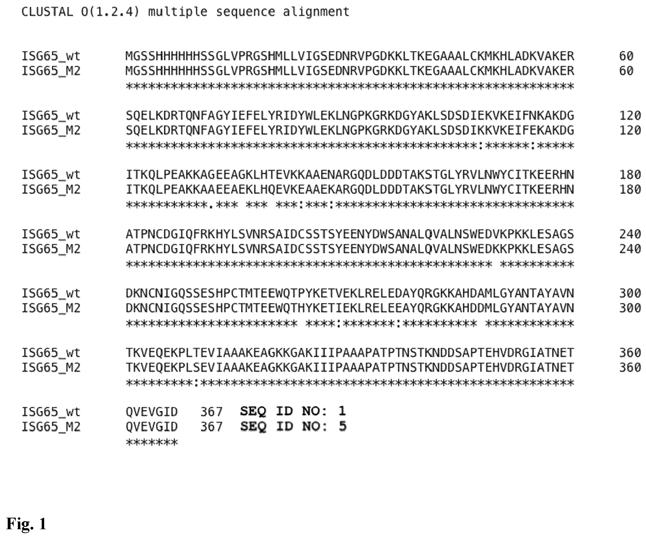

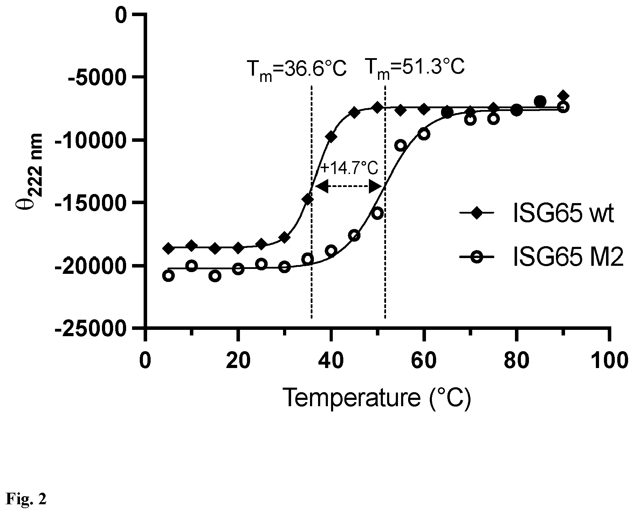

The present invention provides a new ISG65-derived protein exhibiting increased thermostability, The new protein carries several mutations (Fig. 1) and shows a significant and unexpected increase in the melting temperature (ISG65 M2 by 14.7 °C), compared to the native ISG65 sequence (Fig. 2).

Native extracellular domain of ISG65 has the amino acid sequence SEQ ID NO: 1:

```
MMKYLLVFAIIATRIPVLLVIGSEDNRVPGDKKLTKEGAAALCKMKHLADKVAKERSQEL
KDRTQNFAGYIEFELYRIDYWLEKLNGPKGRKDGYAKLSDSDIEKVKEIFNKAKDGITKQ
LPEAKKAGEEAGKLHTEVKKAAENARGQDLDDDTAKSTGLYRVLNWYCITKEERHNATPN
CDGIQFRKHYLSVNRSAIDCSSTSYEENYDWSANALQVALNSWEDVKPKKLESAGSDKNC
NIGQSSESHPCTMTEEWQTPYKETVEKLRELEDAYQRGKKAHDAMLGYANTAYAVNTKVE
QEKPLTEVIAAAKEAGKKGAKIIIPAAAPATPTNSTKNDDSAPTEHVDRGIATNETQVEV
GIDADFDSLLDATEAAEVTRRHQRTAM.
```

The extracellular domain of ISG65 consists mainly of an approx. 80 Å (8 nm) long 3-helix bundle. Extensive loop structures stabilised by disulphide bonds establish the head domain which is located near the N-terminus of the protein and membrane-distal in the mature, membrane-embedded protein. The membrane-proximal parts of bundle helices 1 and 3 contain the majority of C3-TED interacting residues. Another interaction site is located in a loop connecting helices 2 and 3, just C-terminal of a short $3_{10}$-helix.

The three interaction sites are underlined in SEQ ID NO: 1 and depicted in bold in sequences SEQ ID NO: 5-8, and are the following:

> site 1: 70-YIEFELYRIDYWLEKLNGPKGRKDGYAK-97 (SEQ ID NO: 2)
> site 2: 210-DWSA-213 (SEQ ID NO: 3)
> site 3: 290-NTAYAVNTKVEQE-302 (SEQ ID NO: 4)

Within the interaction sites, the identified crucial interface residues are: Tyr70, Phe73, Arg77, Trp81, Lys97; Trp211; Asn290, Tyr293, Thr297, Glu302.

The sequence of the extracellular domain of the thermostable mutant of ISG65 used in the experiments presented in this application was SEQ ID NO: 10, which consists of SEQ ID NO: 5, with affinity tag SEQ ID NO: 11. This sequence is referred to herein as ISG65 M2.

```
LLVIGSEDNRVPGDKKLTKEGAAALCKMKHLADKVAKERSQELKDRTQNFAGYIEFELYRIDYWLEKL

NGPKGRKDGYAKLSDSDIKKVKEIFEKAKDGITKQLPEAKKAAEEAEKLHQEVKEAAEKARGQDLDDD

TAKSTGLYRVLNWYCITKEERHNATPNCDGIQFRKHYLSVNRSAIDCSSTSYEENYDWSANALQVALN

SWEDKKPKKLESAGSDKNCNIGQSSESHPCTMTEEWQTHYKETIEKLRELEEAYQRGKKAHDDMLGYA

NTAYAVNTKVEQEKPLSEVIAAAKEAGKKGAKIIIPAAAPATPTNSTKNDDSAPTEHVDRGIATNETQ

VEVGID (SEQ ID NO: 5)
```

MGSSHHHHHHSSGLVPRGSHM (SEQ ID NO: 11)

Residues mutated in the thermostable ISG65 mutant are: Glu104Lys, Asn111Glu, Gly128Ala, Gly132Glu, Thr136Gln, Lys140Glu, Asn144Lys, Val226Lys, Pro260His, Val265Ile, Asp273Glu, Ala284Asp, Thr306Ser
These residues are underlined in the sequences SEQ ID NO: 5-8.

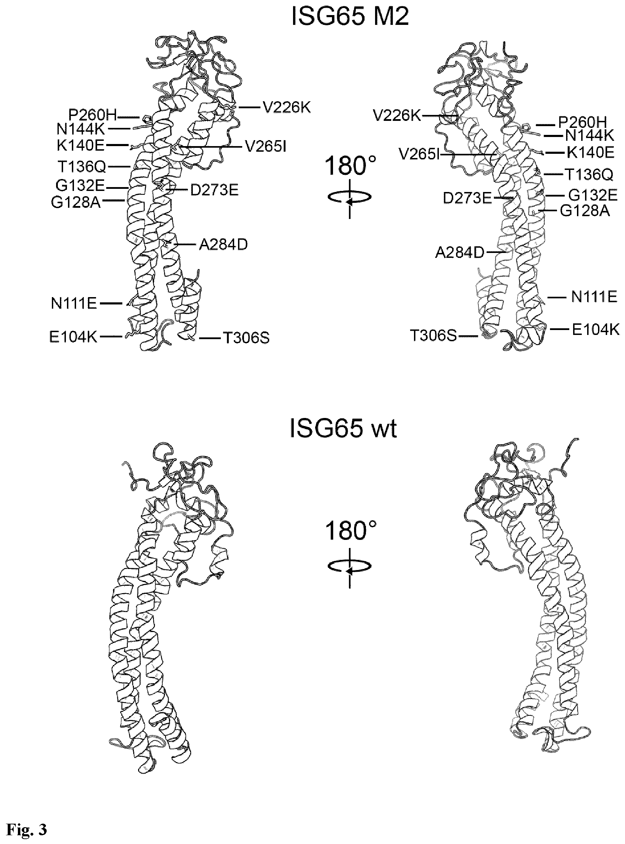

Overall, 13 amino acids were mutated in the new thermostable mutant in comparison to wild type ISG65. Their positions are marked in a sequence alignment between ISG65 M2 and wild type ISG65 (Fig. 1) and were mapped onto a structural model of ISG65 M2 (Fig. 3).

The term "thermostable ISG65 mutant" or "thermostable mutant of ISG65" herein refers to proteins having the length of up to 500 amino acid residues and containing an amino acid sequence having at least 98% identity, preferably at least 99% identity, to the sequence SEQ ID NO: 6, wherein the bolded and underlined regions are conserved:

**YIEFELYRIDYWLEKLNGPKGRKDGYAK**LSDSDIKKVKEIFEKAKDGITKQLPEAKKAAEEAEKLHQE

VKEAAEKARGQDLDDDTAKSTGLYRVLNWYCITKEERHNATPNCDGIQFRKHYLSVNRSAIDCSSTSY

EENY**DWSA**NALQVALNSWEDKKPKKLESAGSDKNCNIGQSSESHPCTMTEEWQTHYKETIEKLRELEE

AYQRGKKAHDDMLGYA**NTAYAVNTKVEQE**KPLS (SEQ ID NO: 6).

In some embodiments, preferred embodiments of "thermostable ISG65 mutant" or "thermostable mutant of ISG65" are sequences containing or consisting of sequences SEQ ID NO: 5, 7 and 8.

The term "identity" refers to the number of identical residues over the total length of the reference sequence. The reference alignment is the CLUSTAL 0(1.2.4) multiple sequence alignment (https://www.ebi.ac.uk/Tools/msa/clustalo/).

The longest, truncated sequence of the thermostable ISG65 mutant that can be produced recombinantly is SEQ ID NO: 7:

MMKYLLVFAIIATRIPVLLVIGSEDNRVPGDKKLTKEGAAALCKMKHLADKVAKERSQELKDRTQNFA

G**YIEFELYRIDYWLEKLNGPKGRKDGYAK**LSDSDI<u>K</u>KVKEI<u>F</u>EKAKDGITKQLPEAKKA<u>A</u>EEA<u>E</u>KLH<u>Q</u>

EVKE<u>A</u>AE<u>K</u>ARGQDLDDDTAKSTGLYRVLNWYCITKEERHNATPNCDGIQFRKHYLSVNRSAIDCSSTS

YEENY**DWSA**NALQVALNSWED<u>K</u>KPKKLESAGSDKNCNIGQSSESHPCTMTEEWQT<u>H</u>YKET<u>I</u>EKLRELE

<u>E</u>AYQRGKKAHD<u>D</u>MLGYA**NTAYAVNTKVEQE**KPL<u>S</u>EVIAAAKEAGKKGAKIIIPAAAPATPTNSTKNDD

SAPTEHVDRGIATNETQVEVGIDAD.

In some embodiments, the invention includes proteins having the length of up to 500 amino acid residues and containing an amino acid sequence having at least 98% identity, preferably at least 99% identity, to the sequence SEQ ID NO: 7, wherein the bolded and underlined regions are conserved.

In some embodiments, a sequence of the extracellular domain of the thermostable ISG65 mutant that can be produced recombinantly is SEQ ID NO: 8:

SEDNRVPGDKKLTKEGAAALCKMKHLADKVAKERSQELKDRTQNFAG**YIEFELYRIDYWLEKLNGPKG**

**RKDGYAK**LSDSDI<u>K</u>KVKEI<u>F</u>EKAKDGITKQLPEAKKA<u>A</u>EEA<u>E</u>KLH<u>Q</u>EVKE<u>A</u>AE<u>K</u>ARGQDLDDDTAKST

GLYRVLNWYCITKEERHNATPNCDGIQFRKHYLSVNRSAIDCSSTSYEENY**DWSA**NALQVALNSWED<u>K</u>

KPKKLESAGSDKNCNIGQSSESHPCTMTEEWQT<u>H</u>YKET<u>I</u>EKLRELE<u>E</u>AYQRGKKAHD<u>D</u>MLGYA**NTAYA**

**VNTKVEQE**KPL<u>S</u>EV

In some embodiments, the invention includes proteins having the length of up to 500 amino acid residues and containing an amino acid sequence having at least 98% identity, preferably at least 99% identity, to the sequence SEQ ID NO: 8, wherein the bolded and underlined regions are conserved. The length of the thermostable ISG65 mutant is up to 500 amino acid residues, more preferably up to 450 amino acid residues, in some embodiments up to 400 amino acid residues. The protein may be extended on one or both termini of the herein shown sequence. The extension sequence may contain, for example, an affinity tag, or a sequence with at least 90% identity to the corresponding ISG65 region.

Affinity tags are known in the art, and include, for example: ALFA-tag, AviTag, C-tag, polyglutamare tag, polyarginine tag, E-tag, FLAG-tag, HA-tag, His-tag, Myc-tag, NE-tag, Rho1D4-tag, S-tag, Softag1, Softag3, Spot-tag, Strep-tag, T7-tag, TC tag, Ty tag, V5 tag, VSV-tag, Xpress tag, Isopeptag, SpyTag, Snoop Tag, DogTag, or SdyTag.

The invention further provides the thermostable ISG65 mutant for use as a medicament, in particular for use in the treatment of complement dysregulation diseases. "Dysregulation" herein refers in particular to excessive activation.

More specifically, the invention provides the thermostable ISG65 mutant for use in the treatment of a disease selected from atypical hemolytic uremic syndrome (aHUS), C3 glomerulopathy (C3G), paroxysmal nocturnal hemoglobinuria (PNH), autoimmune diseases, Alzheimer's syndrome, schizophrenia, angioedema, macular degeneration, and Crohn's disease.

The present invention further includes a pharmaceutical formulation containing the thermostable ISG65 mutant as described herein and at least one pharmaceutically acceptable excipient. Suitable pharmaceutically acceptable excipients include water, buffer, stabilizer, surfactant, binder, filler and glidant.

Brief Description of Drawings

Fig. 1. Multiple sequence alignment of ISG65 wt and the mutated protein ISG65 M2. In total, 13 residues are changed in ISG65 M2 compared to ISG65 wt. Residues are compared based on their physio-chemical proletaries and declared as identical (*), highly similar (:), similar (.) and dissimilar ( ). The alignment was generated using the software Clustal O(mega) (https://www.ebi.ac.uk/Tools/msa/clustalo/).

Fig. 2. Melting curves of ISG65 wt and ISG65 M2. The melting temperature ($T_m$) of ISG65 M2 is increased by 14.7°C compared to ISG65 wt. Curves and melting temperatures were calculated from circular dichroism spectra at 222nm wavelength. The melting temperatures were determined from the inflection points of the sigmoidal unfolding curves. θ, molar ellipticity (deg cm$^2$ dmol$^{-1}$)

**Fig. 3.** Structural comparison of ISG65 M2 and ISG65 wt in 2 different orientations. Positions of the thermostability improving mutations in ISG65 M2 are indicated. All mutations are located outside the C3 binding site of ISG65. The first letter represents the mutated residue in ISG65 M2, the number represents the position in the protein and the last letter the residue that was mutated in the wt protein. To obtain a better visual overview about the positions of the introduced mutations, the model of ISG65 M2 was computationally predicted using the software AlphaFold2 (top) (Jumper J, Evans R, Pritzel A, et al. Highly accurate protein structure prediction with AlphaFold. Nature. 2021;596(7873):583-589. doi:10.1038/s41586-021-03819-2.) The model of ISG65 wt (bottom) is derived from experimental data (cryo-EM). ISG65 wt and ISG65 M2 are highly similar and can be aligned with an RSMD=2.1 Å (Pymol Align, Schrödinger, L., & DeLano, W. (2020). PyMOL. Retrieved from http://www.pymol.org/pymol). This implies that the indicated mutations are unlikely to change the overall fold and the functional properties of ISG65. RMSD, root-mean-square deviation of atomic positions.

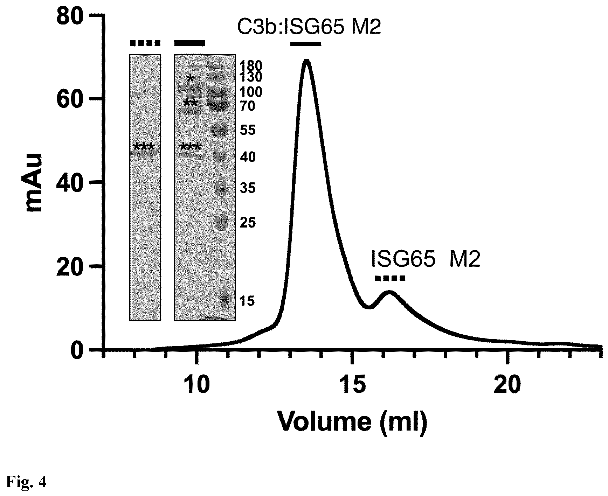

**Fig. 4.** Chromatogram showing purification of the C3b: ISG65 M2 complex (~220 kDa) using size exclusion chromatography on Superdex S200 10/300. Complex formation of ISG65 M2 with C3b was confirmed by analysing fractions underneath the main peak (marked with horizontal bar) on a reducing SDS gel. *C3b alpha-chain, **C3b beta-chain, ***ISG65.

Examples of carrying out the invention

**Cloning, expression and purification of ISG65 M2**

A DNA fragment (Genewiz) encoding amino acids (aa) 18-363 of ISG65 from *T. b. gambiense* (Tbg.972.2.1600) including ISG65 M2 specific mutations was codon-optimised for bacterial expression and cloned into the pET15b plasmid using Gibson assembly method (New England Biolabs). For recombinant expression in *E. coli* T7 shuffle cells (New England Biolabs), a plasmid encoding an N-terminal hexa-histidine-tag (His-ISG65$_{18-363}$) was generated. ISG65 M2 was produced overnight at 22°C after induction of protein expression with 1 mM Isopropyl-b-D-thiogalactopyranoside (IPTG). Soluble protein was purified from bacterial lysate by immobilised metal-affinity chromatography (IMAC) using nickel-nitrilotriacetic acid (NTA) beads (Qiagen) and gravity-flow columns (Bio-Rad). After IMAC, the purified proteins were concentrated using Amicon Ultra centrifugal filters (Merck Millipore) and subjected to size-exclusion chromatography using a Superdex 200 Increase 10/300 GL (GE Healthcare) equilibrated with 20 mM HEPES (pH 7.5) and 150 mM NaCl.

The DNA sequence of the expression construct was SEQ ID NO: 9

```
ATGGGCAGCAGCCATCATCATCATCATCACAGCAGCGGCCTGGTGCCGCGCGGCAGCCATATGCTGCT
GGTGATTGGCAGCGAAGATAACCGCGTGCCGGGCGATAAAAACTGACCAAAGAAGGCGCGGCGGCGC
TGTGCAAAATGAAACATCTGGCGGATAAAGTGGCGAAAGAACGCAGCCAAGAACTGAAAGATCGCACG
CAGAACTTTGCGGGCTATATTGAATTTGAACTGTATCGCATTGATTATTGGCTGGAAAAACTGAACGG
CCCGAAAGGCCGCAAAGATGGCTATGCGAAACTGAGCGATAGCGATATTAAAAAAGTGAAAGAAATTT
TTGAAAAAGCGAAAGATGGCATTACCAAACAGCTGCCGGAAGCGAAAAAAGCGGCGGAAGAAGCGGAA
AAACTGCATCAAGAAGTGAAAGAAGCGGCGGAAAAAGCGCGCGGCCAAGATCTGGATGATGATACCGC
GAAAAGCACCGGCCTGTATCGCGTGCTGAACTGGTATTGCATTACCAAAGAAGAACGCCATAACGCGA
CCCCGAACTGCGATGGCATTCAGTTTCGCAAACATTATCTGAGCGTGAACCGCAGCGCGATTGATTGC
AGCAGCACGAGCTATGAAGAAACTATGATTGGAGCGCGAACGCGCTGCAAGTGGCGCTGAACAGCTG
GGAAGATAAAAACCGAAAAACTGGAAAGCGCGGGCAGCGATAAAAACTGCAACATTGGTCAGAGCA
GCGAAAGCCATCCGTGCACCATGACCGAAGAATGGCAGACCCATTATAAAGAAACCATTGAAAAACTG
CGCGAACTGGAAGAAGCGTATCAGCGCGGCAAAAAAGCGCATGATGATATGCTGGGCTATGCGAACAC
CGCGTATGCGGTGAACACCAAAGTGGAACAAGAAAAACCGCTGAGCGAAGTGATTGCGGCGGCGAAAG
AAGCGGGCAAAAAAGGCGCGAAAATTATTATTCCGGCGGCGGCGCCGGCGACCCCGACCAACAGCACC
AAAAACGATGATAGCGCGCCGACCGAACATGTGGATCGCGGCATTGCGACCAACGAAACCCAAGTGGA
AGTGGGCATTGATTAA
```

The translated sequence, including the affinity tag, was SEQ ID NO: 10

```
MGSSHHHHHHSSGLVPRGSHMLLVIGSEDNRVPGDKKLTKEGAAALCKMKHLADKVAKERSQELKDRT
QNFAGYIEFELYRIDYWLEKLNGPKGRKDGYAKLSDSDIKKVKEIFEKAKDGITKQLPEAKKAAEEAE
KLHQEVKEAAEKARGQDLDDDTAKSTGLYRVLNWYCITKEERHNATPNCDGIQFRKHYLSVNRSAIDC
SSTSYEENYDWSANALQVALNSWEDKKPKKLESAGSDKNCNIGQSSESHPCTMTEEWQTHYKETIEKL
RELEEAYQRGKKAHDDMLGYANTAYAVNTKVEQEKPLSEVIAAAKEAGKKGAKIIIPAAAPATPTNST
KNDDSAPTEHVDRGIATNETQVEVGID
```

**ISG65 M2 binds to C3b**

All thermostability increasing mutations in ISG65 M2 were introduced outside the C3b binding interfaces. In order to confirm C3b binding in solution, analytical size exclusion chromatography was carried out (Fig. 4). ISG65 M2 was hereby mixed and incubated with C3b at 3-fold molar excess, concentrated, injected onto a gel-filtration column (Superdex 200 10/300) and the eluate fractionated. Analysis of protein fractions underneath the main UV peaks by SDS-PAGE revealed that free ISG65 M2 has been depleted and elutes earlier, together with C3b, indicating a specific, high-affinity interaction between both proteins (Fig. 4).

**Circular dichroism spectroscopy**

In order to compare the differences in melting temperatures ($T_m$) between ISG65 wt and ISG65 M2, far-UV CD experiments were carried out on a Jasco J-1500 spectropolarimeter with a 0.2 mm path cell. ISG65 was dissolved in 10 mM HEPES pH 7.5, 150 mM NaF at a concentration of 0.4 mg/ml.

Spectra were recorded between 195 and 260 nm wavelength at an acquisition speed of 10 nm/min and corrected for buffer absorption. For calculation of melting curves at 222 nm, spectra were recorded every 5 degrees between 5 °C and 80 °C with a slope of 0.16 °C/min. During measurements, the temperature was kept constant. The raw CD data (ellipticity θ in mdeg) were normalized for the protein concentration and for the number of residues, according to the equation below, yielding the mean residue ellipticity ($[\theta]$ in deg cm$^2$ mol$^{-1}$), where *MM, n, C*, and *l* denote the molecular mass (Da), the number of amino acids, the concentration (mg/mL), and the cuvette path length (cm), respectively (Fig. 2).

$$[\theta] = \frac{\theta \cdot MM}{n \cdot C \cdot l}$$

**Claims**

1. Thermostable ISG65 mutant having the length of up to 500 amino acid residues and containing an amino acid sequence having at least 98% identity, preferably at least 99% identity, to the sequence SEQ ID NO: 6, wherein the bolded regions and the underlined amino acids are conserved

   **YIEFELYRIDYWLEKLNGPKGRKDGYAK**LSDSDI<u>K</u>KVKEIFE<u>K</u>AKDGITKQLPEAKKA<u>A</u>EEA<u>E</u>KLH<u>Q</u>EVK<u>E</u>AAEK

   ARGQDLDDDTAKSTGLYRVLNWYCITKEERHNATPNCDGIQFRKHYLSVNRSAIDCSSTSYEENY**DWSA**NALQVA

   LNSWED<u>K</u>KPKKLESAGSDKNCNIGQSSESHPCTMTEEWQT<u>H</u>YKET<u>I</u>EKLRELE<u>E</u>AYQRGKKAHDD<u>M</u>LGYA**NTAYA**

   **VNTKVEQE**KPL<u>S</u>  (SEQ ID NO: 6).

2. Thermostable ISG65 mutant according to claim 1, having the length of up to 500 amino acid residues and containing an amino acid sequence having at least 98% identity, preferably at least 99% identity, to the sequence SEQ ID NO: 8, wherein the bolded regions and the underlined amino acids are conserved

   SEDNRVPGDKKLTKEGAAALCKMKHLADKVAKERSQELKDRTQNFAG**YIEFELYRIDYWLEKLNGPKGRKDGYAK**

   LSDSDI<u>K</u>KVKEIFE<u>K</u>AKDGITKQLPEAKKA<u>A</u>EEA<u>E</u>KLH<u>Q</u>EVK<u>E</u>AAE<u>K</u>ARGQDLDDDTAKSTGLYRVLNWYCITKE

   ERHNATPNCDGIQFRKHYLSVNRSAIDCSSTSYEENY**DWSA**NALQVALNSWED<u>K</u>KPKKLESAGSDKNCNIGQSSE

   SHPCTMTEEWQT<u>H</u>YKET<u>I</u>EKLRELE<u>E</u>AYQRGKKAHD<u>DM</u>LGYA**NTAYAVNTKVEQE**KPLSEV  (SEQ ID NO: 8).

3. Thermostable ISG65 mutant according to claim 1, having the amino acid sequence

SEDNRVPGDKKLTKEGAAALCKMKHLADKVAKERSQELKDRTQNFAG**YIEFELYRIDYWLEKLNGPKGRKDGYAK**LSDSDIKKVKEIFEKAKDGITKQLPEAKKAAEEAEKLHQEVKEAAEKARGQDLDDDTAKSTGLYRVLNWYCITKEERHNATPNCDGIQFRKHYLSVNRSAIDCSSTSYEENY**DWSA**NALQVALNSWEDKKPKKLESAGSDKNCNIGQSSESHPCTMTEEWQTHYKETIEKLRELEEAYQRGKKAHDDMLGYA**NTAYAVNTKVEQE**KPLSEV  (SEQ ID NO: 8).

4. Thermostable ISG65 mutant according to claim 1, having the length of up to 500 amino acid residues and containing an amino acid sequence having at least 98% identity, preferably at least 99% identity, to the sequence SEQ ID NO: 7, wherein the bolded regions and the underlined amino acids are conserved

MMKYLLVFAIIATRIPVLLVIGSEDNRVPGDKKLTKEGAAALCKMKHLADKVAKERSQELKDRTQNFAG**YIEFEL**
**YRIDYWLEKLNGPKGRKDGYAK**LSDSDIKKVKEIFEKAKDGITKQLPEAKKAAEEAEKLHQEVKEAAEKARGQDL
DDDTAKSTGLYRVLNWYCITKEERHNATPNCDGIQFRKHYLSVNRSAIDCSSTSYEENY**DWSA**NALQVALNSWED
KKPKKLESAGSDKNCNIGQSSESHPCTMTEEWQTHYKETIEKLRELEEAYQRGKKAHDDMLGYA**NTAYAVNTKVE**
**QE**KPLSEVIAAAKEAGKKGAKIIIPAAAPATPTNSTKNDDSAPTEHVDRGIATNETQVEVGIDAD  (SEQ ID NO:
7).

5. Thermostable ISG65 mutant according to claim 1, having the amino acid sequence

MMKYLLVFAIIATRIPVLLVIGSEDNRVPGDKKLTKEGAAALCKMKHLADKVAKERSQELKDRTQNFAG**YIEFEL**
**YRIDYWLEKLNGPKGRKDGYAK**LSDSDIKKVKEIFEKAKDGITKQLPEAKKAAEEAEKLHQEVKEAAEKARGQDL
DDDTAKSTGLYRVLNWYCITKEERHNATPNCDGIQFRKHYLSVNRSAIDCSSTSYEENY**DWSA**NALQVALNSWED
KKPKKLESAGSDKNCNIGQSSESHPCTMTEEWQTHYKETIEKLRELEEAYQRGKKAHDDMLGYA**NTAYAVNTKVE**
**QE**KPLSEVIAAAKEAGKKGAKIIIPAAAPATPTNSTKNDDSAPTEHVDRGIATNETQVEVGIDAD  (SEQ ID NO:
7).

6. Thermostable ISG65 mutant according to claim 1, having the length of up to 500 amino acid residues and containing an amino acid sequence having at least 98% identity, preferably at least 99% identity, to the sequence SEQ ID NO: 5, wherein the bolded regions and the underlined amino acids are conserved

LLVIGSEDNRVPGDKKLTKEGAAALCKMKHLADKVAKERSQELKDRTQNFAG**YIEFELYRIDYWLEKLNGPKGRK**
**DGYAK**LSDSDIKKVKEIFEKAKDGITKQLPEAKKAAEEAEKLHQEVKEAAEKARGQDLDDDTAKSTGLYRVLNWY
CITKEERHNATPNCDGIQFRKHYLSVNRSAIDCSSTSYEENY**DWSA**NALQVALNSWEDKKPKKLESAGSDKNCNI
GQSSESHPCTMTEEWQTHYKETIEKLRELEEAYQRGKKAHDDMLGYA**NTAYAVNTKVEQE**KPLSEVIAAAKEAGK
KGAKIIIPAAAPATPTNSTKNDDSAPTEHVDRGIATNETQVEVGID  (SEQ ID NO: 5).

7. Thermostable ISG65 mutant according to claim 1, having the amino acid sequence

LLVIGSEDNRVPGDKKLTKEGAAALCKMKHLADKVAKERSQELKDRTQNFAG**YIEFELYRIDYWLEKLNGPKGRK**
**DGYAK**LSDSDIKKVKEIFEKAKDGITKQLPEAKKAAEEAEKLHQEVKEAAEKARGQDLDDDTAKSTGLYRVLNWY
CITKEERHNATPNCDGIQFRKHYLSVNRSAIDCSSTSYEENY**DWSA**NALQVALNSWEDKKPKKLESAGSDKNCNI
GQSSESHPCTMTEEWQTHYKETIEKLRELEEAYQRGKKAHDDMLGYA**NTAYAVNTKVEQE**KPLSEVIAAAKEAGK
KGAKIIIPAAAPATPTNSTKNDDSAPTEHVDRGIATNETQVEVGID  (SEQ ID NO: 5).

8. Thermostable ISG65 mutant according to any one of claims 1 to 7 for use as a medicament.

9. Thermostable ISG65 mutant according to any one of claims 1 to 7 for use in the treatment of a complement dysregulation disease.

10. Thermostable ISG65 mutant according to any one of claims 1 to 7 for use in the treatment of a disease selected from atypical hemolytic uremic syndrome (aHUS), C3 glomerulopathy (C3G), paroxysmal nocturnal hemoglobinuria (PNH), autoimmune diseases, Alzheimer's syndrome, schizophrenia, angioedema, macular degeneration, and Crohn's disease.

11. A pharmaceutical formulation containing the thermostable ISG65 mutant according to any one of claims 1 to 7 and at least one pharmaceutically acceptable excipient.

12. The pharmaceutical formulation according to claim 11 wherein the pharmaceutically acceptable excipient is selected from water, buffer, stabilizer, surfactant, binder, filler and glidant.

```
CLUSTAL O(1.2.4) multiple sequence alignment


ISG65_wt    MGSSHHHHHHSSGLVPRGSHMLLVIGSEDNRVPGDKKLTKEGAAALCKMKHLADKVAKER          60
ISG65_M2    MGSSHHHHHHSSGLVPRGSHMLLVIGSEDNRVPGDKKLTKEGAAALCKMKHLADKVAKER          60
            ************************************************************

ISG65_wt    SQELKDRTQNFAGYIEFELYRIDYWLEKLNGPKGRKDGYAKLSDSDIEKVKEIFNKAKDG         120
ISG65_M2    SQELKDRTQNFAGYIEFELYRIDYWLEKLNGPKGRKDGYAKLSDSDIKKVKEIFEKAKDG         120
            ***********************************************:******:*****

ISG65_wt    ITKQLPEAKKAGEEAGKLHTEVKKAAENARGQDLDDDTAKSTGLYRVLNWYCITKEERHN         180
ISG65_M2    ITKQLPEAKKAAEEAEKLHQEVKEAAEKARGQDLDDDTAKSTGLYRVLNWYCITKEERHN         180
            ***********.*** *** ***:***:********************************

ISG65_wt    ATPNCDGIQFRKHYLSVNRSAIDCSSTSYEENYDWSANALQVALNSWEDVKPKKLESAGS         240
ISG65_M2    ATPNCDGIQFRKHYLSVNRSAIDCSSTSYEENYDWSANALQVALNSWEDKKPKKLESAGS         240
            ************************************************ **********

ISG65_wt    DKNCNIGQSSESHPCTMTEEWQTPYKETVEKLRELEDAYQRGKKAHDAMLGYANTAYAVN         300
ISG65_M2    DKNCNIGQSSESHPCTMTEEWQTHYKETIEKLRELEEAYQRGKKAHDDMLGYANTAYAVN         300
            **********************  ****:*******:*********** ***********

ISG65_wt    TKVEQEKPLTEVIAAAKEAGKKGAKIIIPAAAPATPTNSTKNDDSAPTEHVDRGIATNET         360
ISG65_M2    TKVEQEKPLSEVIAAAKEAGKKGAKIIIPAAAPATPTNSTKNDDSAPTEHVDRGIATNET         360
            *********:**************************************************

ISG65_wt    QVEVGID   367   SEQ ID NO: 1
ISG65_M2    QVEVGID   367   SEQ ID NO: 5
            *******
```

**Fig. 1**

Fig. 2

## ISG65 M2

## ISG65 wt

**Fig. 3**

Fig. 4

Europäisches
Patentamt

European
Patent Office

Office européen
des brevets

# EUROPEAN SEARCH REPORT

Application Number

EP 23 15 6132

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE UniProt [Online] <br><br> 24 November 2009 (2009-11-24), "SubName: Full=65 kDa invariant surface glycoprotein, putative {ECO:0000313\|EMBL:CBH09427.1};", XP002808460, retrieved from EBI accession no. UNIPROT:C9ZJ68 Database accession no. C9ZJ68 * sequence * <br> ----- | 1-12 | INV. <br> C07K14/44 <br> A61K38/16 <br> A61P37/00 <br> A61P25/28 |
| Y | MACLEOD OLIVIA J. S. ET AL: "Invariant surface glycoprotein 65 of Trypanosoma brucei is a complement C3 receptor", NATURE COMMUNICATIONS, vol. 13, no. 1, 29 August 2022 (2022-08-29), XP093015177, DOI: 10.1038/s41467-022-32728-9 Retrieved from the Internet: URL:https://www.ncbi.nlm.nih.gov/pmc/articles/PMC9424271/pdf/41467_2022_Article_32728.pdf> * the whole document * <br> ----- | 1-12 | |
| Y | A. M. RISITANO ET AL: "Complement fraction 3 binding on erythrocytes as additional mechanism of disease in paroxysmal nocturnal hemoglobinuria patients treated by eculizumab", BLOOD, vol. 113, no. 17, 23 April 2009 (2009-04-23), pages 4094-4100, XP055053426, ISSN: 0006-4971, DOI: 10.1182/blood-2008-11-189944 * the whole document * <br> ----- | 1-12 | |

TECHNICAL FIELDS
SEARCHED (IPC)

C07K
A61K
A61P

-/--

The present search report has been drawn up for all claims

| Place of search | Date of completion of the search | Examiner |
|---|---|---|
| Munich | 18 July 2023 | Young, Craig |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................

& : member of the same patent family, corresponding document

EPO FORM 1503 03.82 (P04C01)

European Patent Office
Europäisches Patentamt
European Patent Office
Office européen des brevets

Application Number

EP 23 15 6132

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DIMITRIOS C MASTELLOS ET AL: "Compstatin: a C3-targeted complement inhibitor reaching its prime for bedside intervention", EUROPEAN JOURNAL OF CLINICAL INVESTIGATION, WILEY-BLACKWELL PUBLISHING LTD, GB, vol. 45, no. 4, 9 March 2015 (2015-03-09), pages 423-440, XP071218069, ISSN: 0014-2972, DOI: 10.1111/ECI.12419 * the whole document * | 1-12 | |
| T | SÜLZEN HAGEN ET AL: "Cryo-EM structures of Trypanosoma brucei gambiense ISG65 with human complement C3 and C3b and their roles in alternative pathway restriction", NATURE COMMUNICATIONS, vol. 14, no. 1, 27 April 2023 (2023-04-27), XP93063852, DOI: 10.1038/s41467-023-37988-7 Retrieved from the Internet: URL:https://www.ncbi.nlm.nih.gov/pmc/articles/PMC10140031/pdf/41467_2023_Article_37988.pdf> * the whole document * | | |

TECHNICAL FIELDS SEARCHED (IPC)

The present search report has been drawn up for all claims

| Place of search | Date of completion of the search | Examiner |
|---|---|---|
| Munich | 18 July 2023 | Young, Craig |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 22192040

**Non-patent literature cited in the description**

- **TICHACZEK-GOSKA D.** *Adv Clin Exp Med,* January 2012, vol. 21 (1), 105-14
- **WONG E.K.S. ; KAVANGH D.** *Semin Immunopathol,* January 2018, vol. 40 (1), 49-64
- **JUMPER J ; EVANS R ; PRITZEL A et al.** Highly accurate protein structure prediction with AlphaFold. *Nature,* 2021, vol. 596 (7873), 583-589
- **PYMOL ALIGN ; SCHRÖDINGER, L. ; DELANO, W.** *PyMOL,* 2020, http://www.pymol.org/pymol